# EUROPEAN PATENT APPLICATION

(11) **EP 4 306 645 A1**
(43) Date of publication of application: **17.01.2024**
(21) Application number: 22791954.5
(22) Date of filing: 14.04.2022
(51) Int. Cl.: C12N 15/77, C07K 14/34, C12P 13/06

(54) **L-AMINO-ACID-PRODUCING CORYNEBACTERIUM SP. MICROORGANISM, AND METHOD FOR PRODUCING L-AMINO ACIDS BY USING SAME**

(30) Priority: 20.04.2021 KR 20210051233
(71) Applicant: CJ Cheiljedang Corporation, Seoul 04560 (KR)
(72) Inventor: CHOI, Woosung, Seoul 04560 (KR); JANG, Jaewon, Seoul 04560 (KR); BAEK, Mina, Seoul 04560 (KR); LEE, Kwang Woo, Seoul 04560 (KR)
(74) Representative: Icosa
(86) International application number: PCT/KR2022/005385
(87) International publication number: WO 2022/225254

(57) **Abstract**

Provided is a microorganism of the genus *Corynebacterium* producing L-amino acids and a method for producing L-amino acids using the same.

## Description

### [Technical Field]

The present disclosure relates to a microorganism of the genus *Corynebacterium* producing L-amino acids and a method for producing L-amino acids using the same.

### [Background Art]

L-Amino acids have been used in the fields of animal feeds, pharmaceuticals, and cosmetics and have mainly been produced by fermentation using strains belonging to the genus *Corynebacterium* or *Escherichia.* For production of L-amino acids, various studies have been conducted to develop strains producing L-amino acids with high efficiency and fermentation process technology.

Specifically, target substance-specific approaches, such as methods of increasing expression of a gene encoding an enzyme involved in L-amino acid biosynthesis or methods of removing genes unnecessary for the biosynthesis, have mainly been used (US 8048650 B2, *etc*.)*.*

### [Disclosure]

### [Technical Problem]

The present inventors have confirmed enhancement of L-amino acid producing ability of a microorganism of the genus *Corynebacterium* having weakened activity of a protein comprising an amino acid sequence of SEQ ID NO: 1, thereby completing the present disclosure.

### [Technical Solution]

An object of the present disclosure is to provide a microorganism of the genus *Corynebacterium* having weakened activity of a protein comprising an amino acid sequence of SEQ ID NO: 1.

Another object of the present disclosure is to provide a method for producing L-amino acids, the method comprising culturing the microorganism in a culture medium.

Another object of the present disclosure is to provide a composition for producing L-amino acids comprising the microorganism, the culture medium, or a combination thereof.

Another object of the present disclosure is to provide a method for preparing a microorganism of the genus *Corynebacterium* producing L-amino acids, the method comprising weakening activity of a protein comprising an amino acid sequence of SEQ ID NO: 1.

Another object of the present disclosure is to provide a use of a microorganism of the genus *Corynebacterium* having weakened activity of a protein comprising an amino acid sequence of SEQ ID NO: 1 for production of L-amino acids.

### [Advantageous Effects]

L-Amino acids may be produced with high efficiency using the microorganism of the present disclosure.

### [Best Mode]

The present disclosure will be described in detail as follows. Meanwhile, each description and embodiment disclosed in the present disclosure may be applied to different descriptions and embodiments herein. In other words, all combinations of various components disclosed in the present disclosure are included within the scope of the present disclosure. Furthermore, the scope of the present disclosure should not be limited by the specific descriptions provided below.

Those skilled in the art will recognize or be able to ascertain, using no more than routine experimentation, many equivalents to specific embodiments of the present disclosure. Such equivalents are intended to be encompassed in the scope of the present disclosure.

An aspect of the present disclosure provides a microorganism of the genus *Corynebacterium* having weakened activity of a protein comprising an amino acid sequence of SEQ ID NO: 1.

The protein of the present disclosure may have, comprise, or essentially consist of the amino acid sequence as set forth in SEQ ID NO: 1.

As used herein, the term "protein comprising an amino acid sequence of SEQ ID NO: 1" is a protein having ABC transporter permease component activity and may have an NCBI number of NCgI1917.

In the present disclosure, the protein comprising an amino acid sequence of SEQ ID NO: 1 may be an endogenous protein of the strain, without being limited thereto.

The amino acid sequence of the NCgl1917 protein may be obtained from GenBank of the U.S. National Institutes of Health (NIH), which is a known database. For example, information on the amino acid sequence of the NCgl1917 protein may be identified in NCBI Reference Sequence WP_006284211, but is not limited thereto. For example, the NCgl1917 protein may refer to a protein having the ABC transporter permease activity derived from the genus *Corynebacterium,* but is not limited thereto.

In the present disclosure, the amino acid sequence of the NCgl1917 protein may comprise an amino acid sequence having at least 70%, 75%, 80%, 85%, 90%, 95%, 96%, 97%, 98%, 99%, 99.5%, 99.7%, or 99.9% homology or identity with the amino acid sequence set forth in SEQ ID NO: 1. Also, it is obvious to those skilled in that art that any protein having an amino acid sequence comprising deletion, modification, substitution, conservative substitution, or addition of one or several amino acids is within the scope of the present disclosure, as long as the protein has an amino acid sequence retaining the homology or identity and effects corresponding to the protein of the present disclosure.

Examples thereof may include addition or deletion of a sequence to/from the N-terminus, the C-terminus, and/or the middle of the amino acid sequence and naturally occurring mutations, silent mutation or conservative substitution thereof without causing changes in functions of the protein.

As used herein, the term "conservative substitution" refers to a substitution of one amino acid with another amino acid having a similar structural and/or chemical property. The protein may comprise at least one conservative substitution while retaining at least one biological activity. Such amino acid substitution may generally occur based on similarity of polarity, charge, solubility, hydrophobicity, hydrophilicity, and/or amphipathic nature of a residue. For example, electrically charged amino acids with side chains include positively charged (basic) amino acids such as arginine, lysine, and histidine and negatively charged (acidic) amino acids such as glutamic acid and aspartic acid; and uncharged amino acids with side chains include nonpolar amino acids such as glycine, alanine, valine, leucine, isoleucine, methionine, phenylalanine, tryptophan, and proline, and polar or hydrophilic amino acids such as serine, threonine, cysteine, tyrosine, asparagine, and glutamine. Among the amino acids, aromatic amino acids include phenylalanine, tryptophan, and tyrosine.

As used herein, the term "homology" or "identity" refers to relatedness between two given amino acid sequences or nucleotide sequences and may be expressed as a percentage. The terms homology and identity may often be used interchangeably.

Sequence homology or identity of conserved polynucleotides or proteins may be determined by standard alignment algorithm and default gap penalties established by a program may be used together therewith. Substantially, homologous or identical sequences may generally hybridize with each other in whole or in part under moderately or highly stringent conditions. It is obvious that hybridization includes hybridization of a polynucleotide with a polynucleotide comprising a general codon or a codon in consideration of codon degeneracy.

Homology, similarity, or identity between any two polynucleotide or protein sequences may be determined using any computer algorithm known in the art, *e.g.,* "FASTA" program, using default parameters as disclosed in Pearson et al (1988) Proc. Natl. Acad. Sci. USA 85:2444. Alternatively, the homology, similarity, or identity may be determined using the Needleman-Wunsch algorithm (Needleman and Wunsch, 1970, J. Mol. Biol. 48:443-453) as implemented in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST, from the National Center for Biotechnology Information database, or ClustalW.

The homology, similarity, or identity between polynucleotides or proteins may be determined by comparing sequence information using a GAP computer program (*e.g.,* Needleman et al., (1970), J Mol Biol. 48:443) as disclosed by Smith and Waterman, Adv. Appl. Math (1981) 2:482. Briefly, the GAP program defines similarity as the number of aligned symbols (*i.e.,* nucleotides or amino acids) which are similar, divided by the total number of symbols in a shorter of two sequences. Default parameters for the GAP program may include: (1) a binary comparison matrix (containing a value of 1 for identities and 0 for non-identities) and the weighted comparison matrix of Gribskov, et al. (1986), Nucl. Acids Res. 14:6745 as described by Schwartz and Dayhoff, eds., Atlas Of Protein Sequence and Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

In the present disclosure, a polynucleotide encoding the protein comprising the amino acid sequence of SEQ ID NO: 1 may be named *Ncgl1917* gene.

As used herein, the term "polynucleotide" refers to a DNA or RNA strand having a certain minimum length as a polymer of nucleotides in which nucleotide monomers are linked to each other in the form of a long chain by covalent bonds. Specifically, the polynucleotide may be a polynucleotide fragment encoding the protein.

A polynucleotide encoding the protein of the present disclosure may comprise a nucleotide sequence encoding the amino acid sequence as set forth in SEQ ID NO: 1. As an example of the present disclosure, the polynucleotide of the present disclosure may have or comprise a sequence of SEQ ID NO: 2. In addition, the polynucleotide of the present disclosure may comprise or essentially consist of the sequence of SEQ ID NO: 2.

The polynucleotide of the present disclosure may comprise various modifications made in a coding region within a range not to change the amino acid sequence of the polypeptide expressed from the coding region in consideration of codon degeneracy or a codon preferred by a living organism in which the polypeptide is to be expressed.

Specifically, the polynucleotide of the present disclosure may have or comprise a nucleotide sequence having a homology or identity of 70% or more, 75% or more, 80% or more, 85% or more, 90% or more, 95% or more, 96% or more, 97% or more, 98% or more, and less than 100% with the sequence of SEQ ID NO: 2, or may consist of or essentially consist of a nucleotide sequence having a homology or identity of 70% or more, 75% or more, 80% or more, 85% or more, 90% or more, 95% or more, 96% or more, 97% or more, 98% or more, and less than 100% with the sequence of SEQ ID NO: 2, without being limited thereto.

In addition, the polynucleotide may comprise any probe prepared from any known gene sequences, *e.g.,* a nucleotide sequence hybridized with a sequence totally or partially complementary to the above-described nucleotide sequence under stringent conditions, without limitation. The term "stringent conditions" refers to conditions allowing specific hybridization between polynucleotides. Such conditions are disclosed in detail in known documents (J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include allowing hybridization between polynucleotides having a high homology or identity, *e.g.,* a homology or identity of 70% or more, 75% or more, 80% or more, 85% or more, 90% or more, 95% or more, 96% or more, 97% or more, 98% or more, or 99% or more, without allowing hybridization between polynucleotides having a homology or identity lower than the above homologies or identities, or washing once, specifically twice or three times, under conventional washing conditions for Southern hybridization at a salt concentration and temperature of 60°C, 1×SSC, and 0.1% SDS, specifically 60°C, 0.1×SSC,0.1% SDS, and more specifically 68°C, 0.1×SSC,and 0.1% SDS.

Hybridization requires that two nucleic acids have complementary sequences, although bases may mismatch according to the degree of stringency of hybridization. The term "complementary" is used to describe the relationship between bases of nucleotides capable of hybridizing with each other. For example, with respect to DNA, adenine is complementary to thymine and cytosine is complementary to guanine. Thus, the polynucleotide of the present disclosure may comprise not only nucleic acid sequences substantially similar to that of the polynucleotide but also isolated nucleic acid fragments complementary to the entire sequence of the polynucleotide.

Specifically, the polynucleotides having homology or identity with the polynucleotide of the present disclosure may be detected using hybridization conditions including a hybridization process at a Tₘ value of 55°C and the above-described conditions. Also, the Tₘ value may be, but is not limited to, 60°C, 63°C or 65°C, and may be appropriately adjusted by those skilled in the art according to the intended purposes.

An appropriate degree of stringency for hybridization of the polynucleotides may depend on lengths and a degree of complementarity of the polynucleotides and parameters thereof are well known in the art (*e.g.,* J. Sambrook *et al., supra*).

As used herein, the term "microorganism (or strain)" comprises both wild-type microorganisms and microorganisms comprising natural or artificial genetic modification, such as microorganisms having a particular mechanism weakened or enhanced via introduction of an exogenous gene or enhancement or inactivation of an endogenous gene and comprising genetic modification to produce a target protein, protein, or product.

The microorganism of the present disclosure may be: a microorganism having weakened activity of the protein comprising the amino acid sequence of SEQ ID NO: 1 of the present disclosure; a microorganism in which expression of a polynucleotide encoding the protein comprising an amino acid sequence of SEQ ID NO: 1 of the present disclosure is weakened; or a microorganism genetically modified (*e.g.,* recombinant microorganism) to have weakened activity of the protein comprising the amino acid sequence of SEQ ID NO: 1 of the present disclosure by using a vector, without being limited thereto.

For example, the weakened activity of the protein comprising an amino acid sequence of SEQ ID NO: 1 may be weakened expression of the polynucleotide encoding the amino acid sequence of SEQ ID NO: 1 or the polynucleotide of SEQ ID NO: 2. For example, the weakening of the activity of the protein comprising an amino acid sequence of SEQ ID NO: 1 may be achieved by modification of the nucleotide sequence of the polynucleotide, *e.g.,* insertion, deletion, or substitution of nucleotides. However, the embodiment is not limited thereto.

The microorganism of the present disclosure may be a microorganism having L-amino acid producing ability.

The microorganism of the present disclosure may be a microorganism naturally having the L-amino acid producing ability or a microorganism prepared by providing a parent strain unable to produce an L-amino acid with the L-amino acid producing ability with weakened activity of the protein comprisingthe amino acid sequence of SEQ ID NO: 1 of the present disclosure, but is not limited thereto.

For example, the microorganism of the present disclosure may be a strain or microorganism having weakened activity of the protein comprising the amino acid sequence of SEQ ID NO: 1 via transformation using a vector designed to weaken the activity of the protein comprising the amino acid sequence of SEQ ID NO: 1 of the present disclosure. In view of the objects of the present disclosure, the microorganism may be a microorganism having enhanced L-amino acid producing ability, compared to non-modified microorganisms (*e.g.,* naturally occurring wild-type microorganism or microorganism in which the activity of the protein comprising the amino acid sequence of SEQ ID NO: 1 is not modified), by weakening the activity of the protein comprising an amino acid sequence of SEQ ID NO: 1 of the present disclosure in a naturally occurring wild-type microorganism or a L-amino acid-producing microorganism, but is not limited thereto.

For example, the non-modified microorganism, as a strain for comparison in terms of enhancement of the L-amino acid producing ability, may be *Corynebacterium glutamicum* KCCM12502P (KR 10-2126951 B1), KCCM11222P (US 10590446 B2), or KCCM11248P (KR 10-1335789 B1), without being limited thereto.

For example, in the strain having enhanced L-amino acid producing ability, the L-amino acid producing ability may be enhanced by about 1% or more, specifically about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 20.5% or more, about 21% or more, about 21.5% or more, about 22% or more, about 22.5% or more, about 23% or more, about 23.5% or more, about 24% or more, about 24.5% or more, about 25% or more, about 25.5% or more, about 26% or more, about 26.5% or more, about 27% or more, about 27.5% or more, about 28% or more, about 28.5% or more, about 29% or more, about 29.5% or more, about 30% or more, about 30.5% or more, about 31% or more, about 31.5% or more, about 32% or more, about 32.5% or more, about 33% or more, about 33.5% or more, about 34% or more, about 34.5% or more, or about 35% or more, compared to the L-amino acid producing ability of a parent strain before modification or a non-modified microorganism, but the present disclosure is not limited thereto as long as a positive value of increment for the L-amino acid producing ability is obtained compared to the L-amino acid producing ability of the parent strain before modification or the non-modified microorganism. In another example, in a recombinant strain having enhanced L-amino acid producing ability, the L-amino acid producing ability may be enhanced by about 1.1 times or more, about 1.12 times or more, about 1.13 times or more, 1.15 times or more, 1.16 times or more, 1.17 times or more, 1.18 times or more, 1.19 times or more, 1.2 times or more, 1.25 times or more, 1.3 times or more, or 1.35 times or more, compared to the parent strain before modification or the non-modified microorganism, without being limited thereto. The term "about" refers to a range including ±0.5, ±0.4, ±0.3, ±0.2, ±0.1, or the like and includes all numerical values in the range equal to or similar to the numerical value following the term "about", without being limited thereto.

For example, the microorganism having enhanced L-amino acid producing ability according to the present disclosure may have the activity of the protein comprising an amino acid sequence of SEQ ID NO: 1 less than 100%, for example, about 99.9% or less, about 99% or less, about 98% or less, about 97% or less, about 96% or less, about 95% or less, about 90% or less, about 80% or less, about 70% or less, about 60% or less, about 50% or less, about 40% or less, about 30% or less, about 20% or less, about 10% or less, about 5% or less, or about 0%, compared to a parent strain before modification or a non-modified microorganism, but is not limited thereto.

As used herein, the term "non-modified microorganism" does not exclude strains comprising mutation naturally occurring in microorganisms and may refer to a wild-type strain or a natural-type strain, or a strain before being transformed by genetic modification caused by a natural or artificial factor. For example, the non-modified microorganism may refer to a microorganism before the activity of the protein comprising an amino acid sequence of SEQ ID NO: 1 is weakened as described herein. The "non-modified microorganism" may be used interchangeably with "strain before modification", "microorganism before modification", "non-mutated strain", "non-modified strain", "non-mutated microorganism", or "reference microorganism".

As another example of the present disclosure, the microorganism of the genus *Corynebacterium* of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

As used herein, the term "weakening" of the protein is a concept that comprises both reduction and elimination of the activity compared to intrinsic activity. The term "weakening" may be used interchangeably with inactivation, deficiency, down-regulation, decrease, reduction, and attenuation.

The weakening may comprise: a case in which the activity of the protein is reduced or eliminated compared to intrinsic activity processed by the microorganism due to mutation of a polynucleotide encoding the protein or the like; a case in which the activity and/or concentration (expression level) of the protein in cells are lower than those of the wild-type strain due to suppressed expression of a polynucleotide encoding the same or suppressed translation thereof into the protein; a case in which the polynucleotide is not expressed at all; and/or a case in which no activity of the protein is obtained although the polynucleotide is expressed. The term "intrinsic activity" refers to activity of a protein originally possessed by a parent strain before transformation, wile-type, or non-modified microorganism when the microorganism is transformed by genetic modification caused by a natural or artificial factor. The intrinsic activity may also be used interchangeably with "activity before modification". The "inactivation, deficiency, down-regulation, decrease, reduction, and attenuation" of the activity of the protein compared to intrinsic activity means lowering of the activity compared to the activity of the protein originally possessed by a parent strain before transformation or non-modified microorganism.

The weakening of the activity of the protein may be performed by, but not limited to, any method well known in the art, and may be achieved by applying various methods well known in the art (*e.g.,* Nakashima N et al., Bacterial cellular engineering by genome editing and gene silencing. Int J Mol Sci. 2014;15(2):2773-2793, Sambrook et al. Molecular Cloning 2012, *etc.*).

Specifically, the weakening of the protein of the present disclosure may be achieved by:
(1) deleting a gene encoding the protein in whole or in part;
(2) modifying a gene expression regulatory region (or expression regulatory sequence) to reduce expression of a gene encoding the protein;
(3) modifying an amino acid sequence constituting the protein to eliminate or weaken the activity of the protein (*e.g.,* deletion/substitution/addition of at least one amino acid in the amino acid sequence);
(4) modifying a sequence of a gene encoding to the protein to eliminate or weaken the activity of the protein (*e.g*., deletion/substitution/addition of at least one base of a nucleic acid on a base sequence of the gene of the protein to encode a protein modified to eliminate or weaken the activity of the protein;
(5) modifying a base sequence encoding an initiation codon or a 5'-UTR region of a gene transcript encoding the protein;
(6) introducing an antisense oligonucleotide (*e.g.,* antisense RNA) complementarily binding to the gene transcript encoding the protein;
(7) adding a sequence complementary to a Shine-Dalgarno sequence of a gene encoding the protein upstream of the Shine-Dalgarno sequence to form a secondary structure making binding of a ribosome impossible;
(8) adding a promoter for reverse transcription to the 3' terminus of an open reading frame (ORF) of a sequence of a gene encoding the protein (Reverse transcription engineering, RTE); or
(9) any combination of two or more selected from (1) to (8) above, without being limited thereto.

For example, the deletion of the gene encoding the protein in whole or in part described in (1) above may be achieved by deleting the entire polynucleotide encoding an intrinsic target protein in the chromosome or by replacing the polynucleotide with a polynucleotide in which one or several nucleotides are deleted or with a marker gene.

Also, the modification of the expression regulatory region (or expression regulatory sequence) described in (2) above may be achieved by mutation in the expression regulatory region (or expression regulatory sequence) by deletion, insertion, non-conservative or conservative substitution, or any combination thereof or replacement with a sequence having weaker activity. The expression regulatory region comprises a promoter, an operator sequence, a ribosome binding site encoding sequence, and a sequence for regulating termination of transcription and translation, without being limited thereto.

In addition, the modification of the base sequence encoding the initiation codon or the 5'-UTR region of the gene transcript encoding the protein described in (3) above may be achieved by, for example, substituting the base sequence encoding the intrinsic initiation codon with another base sequence encoding an initiation codon with a lower expression level of the protein, without being limited thereto.

In addition, the modification of the amino acid sequence or the nucleotide sequence described in (4) and (5) above may be conducted by inducing mutation in the amino acid sequence of the protein or in the nucleotide sequence encoding the protein by deletion, insertion, non-conservative or conservative substitution, or any combination thereof or by replacing the amino acid sequence or the nucleotide sequence with an amino acid sequence or a nucleotide sequence modified to have weaker activity or no activity, to weaken the activity of the protein, without being limited thereto. For example, expression of the gene may be suppressed or weakened by forming a termination codon by introducing a mutation into the nucleotide sequence. As another example of modification of the nucleotide sequence, expression of the polynucleotide may be weakened or the activity of a protein encoded by the polynucleotide may be weakened by inserting a transposon into the nucleotide sequence thereof, but the embodiment is not limited thereto.

The introduction of an antisense oligonucleotide (*e.g.,* antisense RNA) complementarily binding to the gene transcript encoding the protein described in (6) above may be achieved by a method disclosed in a document (Weintraub, H. et al., Antisense-RNA as a molecular tool for genetic analysis, Reviews - Trends in Genetics, Vol. 1(1) 1986).

The addition of the sequence complementary to the Shine-Dalgarno sequence of the gene encoding the protein upstream of the Shine-Dalgarno sequence to form the secondary structure making binding of a ribosome impossible described in (7) above may make mRNA translation impossible or reduce speed.

The addition of the promoter for reverse transcription to the 3' terminus of the open reading frame (ORF) of the sequence of the gene encoding the protein (Reverse transcription engineering, RTE) described in (8) above may weaken the activity by forming an antisense nucleotide complementary to the gene transcript encoding the protein.

As used herein, the term "vector" may comprise a DNA construct containing a base sequence of a polynucleotide encoding the target polypeptide and operably linked to a suitable expression regulatory region (expression regulatory sequence) so as to be able to express the target polypeptide in a suitable host cell. The expression regulatory region may comprise a promoter capable of initiating transcription, any operator sequence for regulating the transcription, a sequence encoding a suitable mRNA ribosome binding site, and a sequence for regulating termination of transcription and translation. When a suitable host cell is transformed with the vector, the vector may replicate or function independently from the host genome or may be integrated into genome thereof.

The vector used in the present disclosure is not particularly limited, and any vector known in the art may be used. Examples of conventional vectors may comprise a natural or recombinant plasmid, cosmid, virus and bacteriophage. For example, pWE15, M13, MBL3, MBL4, IXII, ASHII, APII, t10, t11, Charon4A, Charon21A, *etc.* may be used as a phage vector or a cosmid vector. As a plasmid vector, pDZ-based, pBR-based, pUC-based, pBluescriptll-based, pGEM-based, pTZ-based, pCL-based, pET-based vectors, *etc.* may be used. Specifically, pDZ, pDC, pDCM2, pACYC177, pACYC184, pCL, pECCG117, pUC19, pBR322, pMW118, pCC1BAC vectors, *etc.* may be used.

For example, a polynucleotide encoding a target polypeptide may be inserted into the chromosome by using a vector for chromosomal insertion into cells. The insertion of the polynucleotide into the chromosome may be performed by any method known in the art, for example, homologous recombination, without being limited thereto. The polynucleotide may further comprise a selection marker to confirm chromosomal insertion. The selection marker is used to select cells that are transformed with the vector, that is, to confirm insertion of desired nucleic acid molecules, and markers providing selectable phenotypes, such as drug resistance, nutrient requirement, resistance to cytotoxic agents, or surface expression of polypeptide may be used. Only cells expressing the selection marker are able to survive or to show different phenotypes under the environment treated with a selective agent, and thus the transformed cells may be selected.

As used herein, the term "transformation" refers to a process of introducing a vector comprising a polynucleotide encoding a target polypeptide into a host cell or microorganism in such a way that the polypeptide encoded by the polynucleotide is expressed in the host cell. The transformed polynucleotide may be either in a form inserted into the chromosome of the host cell or in a form located outside the chromosome as long as the polypeptide is expressed in the host cell. In addition, the polynucleotide comprises DNA and RNA encoding the target polypeptide. The polynucleotide may be introduced into the host cell in any form as long as the polynucleotide is introduced into the host cell and expressed therein. For example, the polynucleotide may be introduced into the host cell in the form of an expression cassette that is a gene construct comprising all of the essential elements required for self-replication. The expression cassette may generally comprise a promoter operably linked to the polynucleotide, a transcription termination signal, a ribosome binding site, and a translation termination signal. The expression cassette may be in the form of a self-replicable expression vector. Also, the polynucleotide may be introduced into the host cell in its original form and operably linked to a sequence required for the expression in the host cell, without being limited thereto.

In addition, as used herein, the term "operably linked" means a functional linkage between a nucleotide sequence encoding a target protein and a promoter sequence which initiates and mediates transcription of the polynucleotide.

In the microorganism of the present disclosure, modification of the polynucleotide in whole or in part may be induced by (a) homologous recombination using a vector for chromosomal insertion in the microorganism or genome editing using an engineered nuclease (*e.g.,* CRISPR-Cas9) and/or (b) treatment with light such as UV rays and radioactive rays and/or a chemical substance, without being limited thereto. A method of modifying the gene in whole or in part may comprise a DNA recombination technique. For example, a part of or the entire gene may be deleted by inducing homologous recombination by inserting a nucleotide sequence or vector comprising a nucleotide sequence having a homology with a target gene into the microorganism. The inserted nucleotide sequence or vector may comprise a dominant selection marker, without being limited thereto.

Another aspect of the present disclosure provides a method for producing L-amino acids comprising culturing a recombinant microorganism of the genus *Corynebacterium* having weakened activity of the protein comprising an amino acid sequence of SEQ ID NO: 1 in the culture medium.

The protein comprising an amino acid sequence of SEQ ID NO: 1, weakening, and microorganism are as described above.

The microorganism of the genus *Corynebacterium* may be *Corynebacterium glutamicum,* but is not limited thereto, and is as described above.

The L-amino acid may comprise at least one amino acid selected from L-threonine and L-isoleucine, without being limited thereto.

As used herein, the term "culturing" refers to growing the microorganism of the genus *Corynebacterium* of the present disclosure in an appropriately adjusted environment. A culturing process of the present disclosure may be performed using appropriate culture medium and culture conditions well known in the art. The culturing process may be appropriately adjusted by those skilled in the art in accordance with a selected strain. Specifically, the culturing may be performed by a batch culture method, a continuous culture method, and a fed-batch culture method, without being limited thereto.

As used herein, the term "culture medium" refers to a material in which nutrients required for culturing the microorganism of the genus *Corynebacterium* are mixed as main elements and supplies nutrients and growth factors as well as water which are essential for survival and growth. Specifically, although culture media and other culturing conditions for the microorganism of the genus *Corynebacterium* of the present disclosure are not particularly limited as long as the media are commonly used in culturing microorganisms, the microorganism of the genus *Corynebacterium* of the present disclosure may be cultured in an ordinary medium containing appropriate carbon sources, nitrogen sources, phosphorus sources, inorganic compounds, amino acids, and/or vitamins under aerobic conditions while adjusting temperature, pH, and the like.

Specifically, the culture medium for the microorganism of the genus *Corynebacterium* is disclosed in a document ("Manual of Methods for General Bacteriology" by the American Society for Bacteriology (Washington D.C., USA, 1981)).

In the present disclosure, as the carbon sources, carbohydrates such as glucose, sucrose, lactose, fructose, and maltose; sugar alcohols such as mannitol and sorbitol; organic acids such as pyruvic acid, lactic acid, and citric acid; and amino acids such as glutamic acid, methionine, and lysine may be used. In addition, natural organic nutrients such as starch hydrolysates, molasses, blackstrap molasses, rice bran, cassava, sugar cane bagasse, and corn steep liquor may be used, and specifically, carbohydrates such as glucose and sterile pretreated molasses (*i.e.,* molasses converted into reduced sugars) may be used, and suitable amounts of any other carbon sources may also be used without limitation. These carbon sources may be used alone or in combination of at least two thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids, *e.g.,* glutamic acid, methionine, and glutamine, peptone, NZ-amine, meat extract, yeast extract, malt extract, corn steep liquor, casein hydrolysate, fish or degradation products thereof, and defatted soybean cake or degradation products thereof may be used. These nitrogen sources may be used alone or in combination of at least two thereof, without being limited thereto.

As the phosphorus sources, monopotassium phosphate, dipotassium phosphate, or sodium-containing salts corresponding thereto may be used. As inorganic compounds, sodium chloride, calcium chloride, iron chloride, magnesium sulfate, iron sulfate, manganese sulfate, calcium carbonate, and the like may be used. Also, amino acids, vitamins, and/or appropriate precursors may further be included. These components and precursors may be added to the culture medium in a batch or continuous process, without being limited thereto.

In the present disclosure, during the culturing process of the microorganism of the genus *Corynebacterium,* compounds such as ammonium hydroxide, potassium hydroxide, ammonia, phosphoric acid, and sulfuric acid may be properly added to the cultures in order to adjust the pH of the culture medium. Also, a defoaming agent such as fatty acid polyglycol ester may be added during culturing in order to inhibit formation of foams. In addition, oxygen or oxygen-containing gas may be injected into the culture medium to maintain the culture medium in an aerobic condition, or nitrogen, hydrogen, or carbon dioxide gases may be injected into the culture media to maintain the culture in anaerobic and micro-aerobic conditions without injecting any other gases therefor.

In the present disclosure, a culturing temperature may be maintained in the range of 20°C to 45°C, specifically 25°C to 40°C and the culturing may be performed for about 10 to 160 hours, but is not limited thereto.

L-Amino acids produced by the culturing of the present disclosure may be released into the culture medium or remain in the cells.

The method for producing L-amino acids of the present disclosure may further comprise preparing the microorganism of the genus *Corynebacterium,* preparing a culture medium for culturing the microorganism, or any combination thereof (regardless of the order, in any order), for example, before the culturing step.

The method for producing L-amino acids of the present disclosure may further comprise recovering L-amino acids from the culture medium obtained after culturing (culture broth where the culturing is performed) or the microorganism of the genus *Corynebacterium.* The recovering step may further be included after the culturing step.

The recovering may be collecting target L-amino acids using the method for culturing the microorganism of the genus *Corynebacterium, e.g.,* an appropriate method known in the art such as a batch, continuous, or fed-batch method. For example, centrifugation, filtration, treatment with a protein precipitating agent (salting out), extraction, ultrasonic disintegration, ultrafiltration, dialysis, various chromatographic methods such as molecular sieve chromatography (gel permeation), adsorption chromatography, ion-exchange chromatography, and affinity chromatography, high-performance liquid chromatography (HPLC), and any combination thereof may be used, and the target L-amino acids may be recovered from the culture medium or the microorganism using an appropriate method well known in the art.

In addition, the method for producing L-amino acids of the present disclosure may further comprise a purifying step. The purifying step may be performed using an appropriate method well known in the art. In an embodiment, when the method for producing L-amino acids of the present disclosure comprises both the recovering and purifying steps, the recovering and purifying steps may be performed continuously or discontinuously regardless of the order or may be performed simultaneously or as one integrated step, without being limited thereto.

Another aspect of the present disclosure provides a composition for producing L-amino acids comprising: a microorganism of the genus *Corynebacterium* having weakened activity of a protein comprising an amino acid sequence of SEQ ID NO: 1; a culture medium used to culture the same; or a combination thereof.

The protein comprising an amino acid sequence of SEQ ID NO: 1, weakening, culture medium for the microorganism, and L-amino acids are as described above.

The composition of the present disclosure may further comprise any suitable excipient commonly used in compositions for producing amino acids. Examples of the excipient may comprise a preservative, a humectant, a dispersant, a suspending agent, a buffer, a stabilizer, or an isotonic agent, without being limited thereto.

In the composition of the present disclosure, the protein comprising the amino acid sequence of SEQ ID NO: 1, weakening, microorganism, culture medium, and L-amino acids are as described above.

Another aspect of the present disclosure provides a method for preparing a microorganism of the genus *Corynebacterium* producing L-amino acids producing an L-amino acid, the method comprising weakening activity of a protein comprising an amino acid sequence of SEQ ID NO: 1.

The method may further comprise modifying the microorganism to weaken the activity of the protein comprising an amino acid sequence of SEQ ID NO: 1.

The weakening of the protein, L-amino acids, and microorganism are as described above.

Another aspect of the present disclosure provides a use of a microorganism of the genus *Corynebacterium* having weakened activity of a protein comprising an amino acid sequence of SEQ ID NO: 1 for producing L-amino acids.

The protein comprising an amino acid sequence of SEQ ID NO: 1, weakening, microorganism, and L-amino acids are as described above.

### [Mode for Invention]

Hereinafter, the present disclosure will be described in more detail with reference to the following examples and experimental examples. However, the following examples and experimental examples are merely presented to exemplify the present disclosure, and the scope of the present disclosure is not limited thereto.

### Example 1. Construction of Random Mutagenesis Library Using Transposon

In order to obtain strains having enhanced L-threonine producing ability, a random mutagenesis library was constructed as follows. First, *Corynebacterium glutamicum* KCCM12502P (Korean Patent No. 10-2126951), used as a parent strain, was transformed with a plasmid obtained using an EZ-Tn5^{™} <R6Kγori/KAN-2>TnP Transposome^{™} Kit (Epicentre) by an electric-pulse method (Appl. Microbiol. Biotechnol. (1999) 52:541-545) and smeared on a complex plate medium containing kanamycin (25 mg/L), and then about 20,000 colonies were obtained.

### <Complex plate medium (pH 7.0)>

10 g of glucose, 10 g of peptone, 5 g of beef extract, 5 g of yeast extract, 18.5 g of Brain Heart Infusion, 2.5 g of NaCl, 2 g urea, 91 g of sorbitiol, and 20 g of agar (based on 1 L of distilled water).

### Example 2. Screening of Random Mutagenesis Library Using Transposon

About 20,000 colonies obtained in Example 1 were inoculated onto a selective medium containing 300 µL of kanamycin (25 mg/L) and cultured in a 96-deep-well plate at 32°C at 200 rpm for about 24 hours.

### <Selective Medium (pH 8.0)>

10 g of glucose, 5.5 g of (NH₄)₂SO₄, 1.2 g of MgSO₄·7H₂O, 0.8 g of KH₂PO₄, 16.4 g of K₂HPO₄, 100 µg of biotin, 1000 µg of thiamine HCl, 2000 µg of calcium pantothenate, and 2000 µg of nicotinamide (based on 1 L of distilled water).

A ninhydrin method was used to analyze production of L-threonine obtained from the culture broth (Moore, S., Stein, W. H., Photometric ninhydrin method for use in the chromatography of amino acids. J. Biol. Chem. 1948, 176, 367-388). After the culturing was completed, 10 µL of a supernatant of the culture broth was reacted with 190 µL of a ninhydrin solution at 65°C for 30 minutes, and absorbance was measured at 570 nm using a spectrophotometer and compared with that of the control strain, *Corynebacterium glutamicum* KCCM12502P. Then, 60 colonies exhibiting higher absorbance were selected as mutant strains. The other colonies exhibited absorbance similar to or lower than that of the control stain, *Corynebacterium glutamicum* KCCM12502P.

The selected 60 strains were repeatedly cultured in the same manner as described above and reacted with the ninhydrin solution. As a result, the top 10 mutant strains having enhanced L-threonine producing ability compared to that of the parent strain, *Corynebacterium glutamicum* KCCM12502P, were selected and named 12502P-m1, 12502P-m2, 12502P-m3, 12502P-m4, 12502P-m5, 12502P-m6, 12502P-m7, 12502P-m8, 12502P-m9, and 12502P-m10, respectively.

### Example 3. Analysis of L-Threonine Producing Ability of Random Mutant Strains

In order to make a final selection of strains with reproducibly enhanced L-threonine producing ability from the top 10 mutants selected in Example 2, flask culture was carried out using the following culture medium. Production of L-threonine was measured according to the following method. First, each of the strains was inoculated into a 250 mL corner-baffle flask containing 25 mL of a seed medium supplemented with kanamycin (25 mg/L) and cultured with shaking at 30°C for 20 hours at 200 rpm. Subsequently, 1 mL of the seed culture was inoculated into a 250 mL corner-baffle flask containing 24 mL of a production medium supplemented with kanamycin (25 mg/L) and cultured with shaking at 32°C for 72 hours at 200 rpm. Compositions of the seed medium and the production medium are as follows, and the results of the cultured strains are as shown in Table 1.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, and 2 mg of nicotinamide (based on 1 L of distilled water).

### <Production medium (pH 7.0)>

63 g of glucose, 28 g of (NH₄)₂SO₄, 20 g of soybean protein, 14 g of molasses, 1.1 g of KH₂PO₄, 1.2 g of MgSO₄·7H₂O, 1.8 mg of biotin, 9 mg of thiamine HCl, 9 mg of calcium pantothenate, 180 mg of MnSO₄, 200 mg of FeSO₄, 1 mg of ZnSO₄, 1 mg of CuSO₄, and 30 g of CaCO₃ (based on 1 L of distilled water).

**Table 1**

| Concentration of L-threonine produced by 10 selected random mutant strains | | | | | |
|---|---|---|---|---|---|
| | Strain | L-Threonine (g/L) | | | |
| | | Batch 1 | Batch 2 | Batch 3 | Average |
| Control | KCCM12502P | 3.42 | 3.48 | 3.32 | 3.40 |
| 1 | 12502P-m1 | 3.38 | 3.58 | 3.44 | 3.46 |
| 2 | 12502P-m2 | 3.76 | 3.86 | 3.82 | 3.82 |
| 3 | 12502P-m3 | 3.66 | 3.62 | 3.65 | 3.65 |
| 4 | 12502P-m4 | 3.18 | 3.31 | 3.2 | 3.23 |
| 5 | 12502P-m5 | 3.28 | 3.34 | 3.35 | 3.33 |
| 6 | 12502P-m6 | 4.42 | 4.28 | 4.33 | 4.34 |
| 7 | 12502P-m7 | 3.72 | 3.72 | 3.62 | 3.68 |
| 8 | 12502P-m8 | 3.91 | 4.01 | 3.84 | 3.92 |
| 9 | 12502P-m9 | 3.81 | 3.72 | 3.79 | 3.78 |
| 10 | 12502P-m10 | 4.08 | 3.99 | 3.98 | 4.01 |

Among the selected 10 mutant strains, 12502P-m6 was ultimately selected as a strain in which the L-threonine producing ability was significantly enhanced.

### Example 4. Identification of Cause of Enhancement of L-Threonine Producing Ability in Ultimately Selected Strains

In this example, a gene deleted by random insertion of a transposon was tried to be identified in the ultimately selected mutant strains in Example 3. Genomic DNA of the 12502P-m6 was extracted, cleaved, and ligated to transform *E. coli* DH5α, and the strain was smeared onto an LB solid medium containing kanamycin (25 mg/L). After selecting 20 transformed colonies, a plasmid comprising a part of unknown gene was obtained. As a result of analyzing a nucleotide sequence thereof using primers having SEQ ID NOS: 3 and 4 of the EZ-Tn5^{™} <R6Kγori/KAN-2>TnP Transposome^{™} Kit, it was confirmed that the activity of the protein comprising an amino acid sequence of SEQ ID NO: 1 was weakened because a gene comprising a nucleotide sequence of SEQ ID NO: 2 (*Ncgl1917*) was inactivated based on the nucleotide sequences reported to GenBank of the U.S. National Institutes of Health (NIH).

**Table 2**

| Primer | Sequence (5' -> 3') |
|---|---|
| SEQ ID NO: 3 | ACCTACAACAAAGCTCTCATCAACC |
| SEQ ID NO: 4 | CTACCCTGTGGAACACCTACATCT |

### Example 5. Preparation of Recombinant Vector and Strain to Weaken Activity of Protein Comprising Amino Acid Sequence of SEQ ID NO: 1

A recombinant vector capable of deleting the gene having the nucleotide sequence of SEQ ID NO: 2, which was confirmed in Example 4, from the chromosome of the strains belonging to the genus *Corynebacterium* was constructed according to the following method. First, genomic DNA extracted form a WT strain was used as a template, and a restriction enzyme Smal recognition site was inserted into a 5' fragment located 249 bp upstream from *Ncgl1917* gene to synthesize primers of SEQ ID NOS: 5 and 6. In addition, a restriction enzyme Smal recognition site was inserted into a 3' fragment located 410 bp downstream from the *Ncgl1917* gene to synthesize primers of SEQ ID NOS: 7 and 8 (Table 3).

**Table 3**

| Primer | Sequence (5' -> 3') |
|---|---|
| SEQ ID NO: 5 | ACGGCCAGTGAATTCGAGCTCGGTACCCATGCCTAATAA TGAATTCCA |
| SEQ ID NO: 6 | TGGTCCAGCCGAAAAGCCCAAGCACCACCGCAGAGACC CAGAACAGT |
| SEQ ID NO: 7 | GTGGTGCTTGGGCTTTTCGG |
| SEQ ID NO: 8 | TGCAGGTCGACTCTAGAGGATCCCCTCATCGCTTCCTCG ACTTAG |

Specifically, PCR was performed using the chromosomal DNA of the wild-type *Corynebacterium glutamicum* ATCC 13032 as a template (Sambrook et al., Molecular Cloning, a Laboratory Manual (1989), Cold Spring Harbor Laboratories). From the above, DNA fragments of a 249 bp fragment upstream and a 410 bp fragment downstream from a portion, which encodes a protein encoded by the nucleotide sequence of SEQ ID NO: 2, were obtained. In this case, PCR was performed under the following conditions: denaturation at 94°C for 2 minutes, 30 cycles of denaturation at 94°C for 1 minute, annealing at 56°C for 1 minute, and polymerization at 72°C for 40 seconds, and then polymerization at 72°C for 10 minutes. Meanwhile, a pDCM2 vector (KR 10-2020-0136813 A), which was treated with the restriction enzyme Smal and heat-treated at 65°C for 20 minutes, was ligated to the insertion DNA fragment amplified by PCR using an Infusion Cloning Kit, and then *E. coli* DH5α was transformed therewith. The strain was smeared onto an LB solid medium containing kanamycin (25 mg/L). Colonies transformed with a vector into which a target gene was inserted by PCR using primers of SEQ ID NOS: 5 and 8 were selected, and the plasmid was obtained using a known method for extracting plasmids. The plasmid was named pDCM2-ΔNcgl1917. The *Corynebacterium glutamicum* KCCM12502P strain producing threonine was transformed with the prepared vector by an electric-pulse method. The KCCM12502P strain in which the *Ncgl1917* gene was inactivated was named KCCM12502P::Δ1917.

### Example 6. Evaluation of L-Threonine Producing Ability of Strain Having Weakened Activity of Protein Comprising Amino Acid Sequence of SEQ ID NO: 1 Derived from Corynebacterium glutamicum KCCM12502P

In order to analyze L-threonine producing ability of the prepared *Corynebacterium glutamicum* KCCM12502P::Δ1917 strain, a flask culture was performed using the following culture medium. The parent strain, *Corynebacterium glutamicum* KCCM12502P, was used as a control, and production of L-threonine was measured according to the following method. Each of the strains was inoculated into a 250 mL corner-baffle flask containing 25 mL of a seed medium and cultured with shaking at 30°C for 20 hours at 200 rpm. Then, 1 mL of the seed culture was inoculated into a 250 mL corner-baffle flask containing 24 mL of a production medium and cultured with shaking at 32°C for 72 hours at 200 rpm. Compositions of the seed medium and the production medium are as follows, and the results of the cultured strains are as shown in Table 4.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, and 2 mg of nicotinamide (based on 1 L of distilled water).

### <Production medium (pH 7.0)>

63 g of glucose, 28 g of (NH₄)₂SO₄, 20 g of soybean protein, 14 g of molasses, 1.1 g of KH₂PO₄, 1.2 g of MgSO₄·7H₂O, 1.8 mg of biotin, 9 mg of thiamine HCl, 9 mg of calcium pantothenate, 180 mg of MnSO₄, 200 mg of FeSO₄, 1 mg of ZnSO₄, 1 mg of CuSO₄, and 30 g of CaCO₃ (based on 1 L of distilled water).

**Table 4**

| Concentration of L-threonine in parent strain and Ncgl1917-deleted strain | | | | | |
|---|---|---|---|---|---|
| | Strain | L-Threonine (g/L) | | | |
| | | Batch 1 | Batch 2 | Batch 3 | Average |
| Control | KCCM12502P | 3.33 | 3.28 | 3.31 | 3.31 |
| 1 | KCCM12502P::Δ1917 | 4.46 | 4.38 | 4.41 | 4.42 |

As described above, in the case where the gene comprising the nucleotide sequence of SEQ ID NO: 2 was deleted from the L-threonine-producing strain, *Corynebacterium glutamicum* KCCM12502P, it was confirmed that the L-threonine producing ability was enhanced by 35% on average compared to the parent strain.

Therefore, it was confirmed that the L-threonine producing ability may be improved by deleting the gene comprising the nucleotide sequence of SEQ ID NO: 2 from the microorganism of the genus *Corynebacterium.*

Based on the above-described results, it was confirmed that inactivation of the protein comprising the amino acid sequence of SEQ ID NO: 1 in the L-threonine-producing strain is effective on enhancement of the L-threonine producing ability, and the strain KCCM12502P::Δ1917 was named CA09-0907 and deposited at the Korean Culture Center of Microorganisms (KCCM) and designated as Accession No. KCCM12946P on February 1, 2021.

### Example 7. Preparation of Strain Having Weakened Activity of Protein Comprising Amino Acid Sequence of SEQ ID NO: 1 Derived from Corynebacterium glutamicum KCCM11222P and Evaluation of L-Threonine Producing Ability Thereof

In order to identify whether the same effect is obtained in another strain belonging to *Corynebacterium glutamicum* producing L-threonine, a strain was prepared by deleting the gene comprising the nucleotide sequence of SEQ ID NO: 2 from an L-threonine-producing strain, *Corynebacterium glutamicum* KCCM11222P (US 10590446 B2), in the same manner as in Example 5 and named KCCM11222P::Δ1917. In order to analyze the L-threonine producing ability of the prepared *Corynebacterium glutamicum* KCCM11222P::Δ1917 strain, the strain was cultured in the same manner as in Example 6. As a control, the parent strain, *Corynebacterium glutamicum* KCCM11222P, was used. The results of the cultured strain are as shown in Table 5.

**Table 5**

| Concentration of L-threonine produced by parent strain and *Ncgl1917*-deleted strain | | | | | |
|---|---|---|---|---|---|
| | Strain | L-Threonine (g/L) | | | |
| | | Batch 1 | Batch 2 | Batch 3 | Average |
| Control | KCCM11222P | 7.10 | 7.14 | 7.12 | 7.12 |
| 1 | KCCM11222P::Δ1917 | 8.08 | 8.01 | 8.03 | 8.04 |

As described above, in the case where the gene comprising the nucleotide sequence of SEQ ID NO: 2 was deleted from the L-threonine-producing strain, *Corynebacterium glutamicum* KCCM11222P, it was confirmed that the L-threonine producing ability was enhanced by 12% on average.

When combining the results of Examples 5 to 7, it was confirmed that the L-threonine producing ability may be enhanced by weakening the activity of the protein comprising the amino acid sequence of SEQ ID NO: 1 in the microorganism of the genus *Corynebacterium.*

### Example 8. Preparation of Strain Having Weakened Activity of Protein Comprising Amino Acid Sequence of SEQ ID NO: 1 Derived from Corynebacterium glutamicum KCCM11248P and Evaluation of L-Isoleucine Producing Ability Thereof

In order to identify whether the same effect on enhancing L-amino acid producing ability is obtained in the same manner in a strain belonging to the *Corynebacterium glutamicum* producing L-isoleucine, a strain was prepared by deleting the gene comprising the nucleotide sequence of SEQ ID NO: 2 from an L-isoleucine-producing strain, *Corynebacterium glutamicum* KCCM11248P (Korean Patent No. 10-1335789), in the same manner as in Example 5 and named KCCM11248P::Δ1917. In order to analyze the L-isoleucine producing ability of the prepared *Corynebacterium glutamicum* KCCM11248P::Δ1917 strain, a flask culture was performed using the following culture medium. As a control, the parent strain, *Corynebacterium glutamicum* KCCM11248P, was used. Production of L-isoleucine was measured in the following method. First, each of the strains was inoculated into a 250 mL corner-baffle flask containing 25 mL of a seed medium and cultured with shaking at 30°C for 20 hours at 200 rpm. Then, 1 mL of the seed culture was inoculated into a 250 mL corner-baffle flask containing 24 mL of a production medium and cultured with shaking at 32°C for 72 hours at 200 rpm. Compositions of the seed medium and the production medium are as follows, and the results of the cultured strains are as shown in Table 6.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, and 2 mg of nicotinamide (based on 1 L of distilled water)

### <Production medium (pH 7.2)>

100 g of glucose, 5 g of yeast extract, 16 g of (NH₄)₂SO₄, 1 g of KH₂PO₄, 1 g of MgSO₄·7H₂O, 10 mg of FeSO₄·7H₂O, 10 mg of MnSO₄·H₂O, and 200 µg of biotin (based on 1 L of distilled water).

**Table 6**

| Concentration of L-isoleucine produced by parent strain and Ncgl1917-deleted strain | | | | | |
|---|---|---|---|---|---|
| | Strain | L-Isoleucine (g/L) | | | |
| | | Batch 1 | Batch 2 | Batch 3 | Average |
| Control | KCCM11248P | 3.26 | 3.18 | 3.21 | 3.21 |
| 1 | KCCM11248P::Δ1917 | 4.07 | 4.07 | 4.11 | 4.08 |

As described above, it was confirmed that the L-isoleucine producing ability was enhanced by 27% on average when the gene comprising the nucleotide sequence of SEQ ID NO: 2 was deleted compared to the L-isoleucine-producing strain, *Corynebacterium glutamicum* KCCM11248P.

Therefore, it was confirmed that the L-isoleucine producing ability may be enhanced by weakening the activity of the protein comprising the amino acid sequence of SEQ ID NO: 1 in the microorganism of the genus *Corynebacterium.*

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. Therefore, the scope of the disclosure is defined not by the detailed description, but by the claims and their equivalents, and all variations within the scope of the claims and their equivalents are to be construed as being included in the disclosure.

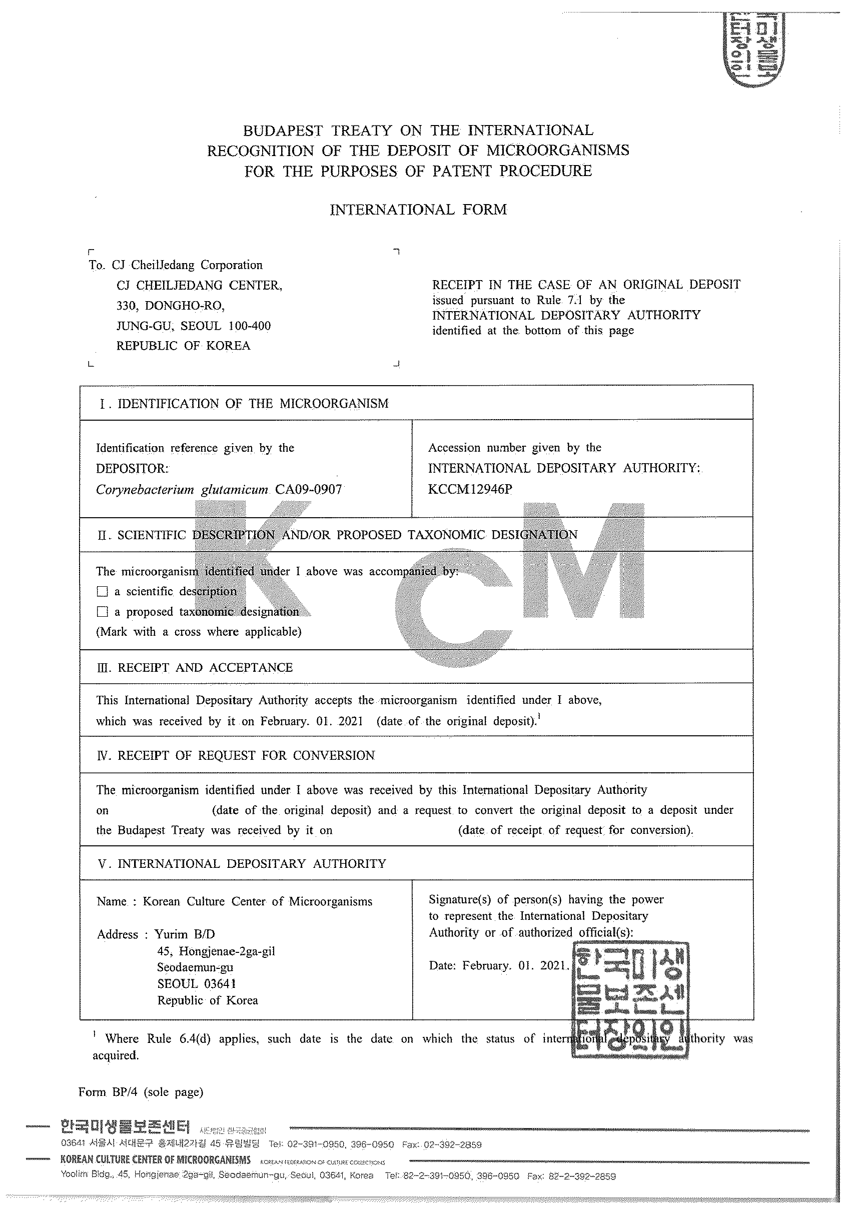

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA09-0907 | Accession number given by the
INTERNATIONAL DEPOSITARY AUTHORITY:
KCCM12946P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on February. 01. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: February. 01. 2021.

¹ Where Rule 6.4(d) applies, such date is the date on which the status of international depositary authority was
acquired.

Form BP/4 (sole page)

한국미생물보존센터 사단법인 한국종균협회
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A microorganism of the genus *Corynebacterium* having weakened activity of a protein comprising an amino acid sequence of SEQ ID NO: 1.

2. The microorganism according to claim 1, wherein the microorganism of the genus *Corynebacterium* is a microorganism producing an L-amino acid.

3. The microorganism according to claim 1, wherein the microorganism of the genus *Corynebacterium* is *Corynebacterium glutamicum.*

4. The microorganism according to claim 2, wherein the L-amino acid comprises at least one selected from L-threonine and L-isoleucine.

5. The microorganism according to claim 1, wherein the microorganism has enhanced L-amino acid producing ability compared to a microorganism in which the activity of the protein comprising an amino acid sequence of SEQ ID NO: 1 is not weakened.

6. A method for producing an L-amino acid, the method comprising culturing the microorganism of the genus *Corynebacterium* according to any one of claims 1 to 5 in a medium.

7. The method according to claim 6, wherein the microorganism of the genus *Corynebacterium* is *Corynebacterium glutamicum.*

8. The method according to claim 6, wherein the L-amino acid comprises at least one selected from L-threonine and L-isoleucine.

9. The method according to claim 6, further comprising recovering the L-amino acid from the microorganism or the medium.

10. A composition for producing an L-amino acid comprising: the microorganism according to any one of claims 1 to 5; a medium used to culture the microorganism; or a combination thereof.

11. The composition according to claim 10, wherein the L-amino acid comprises at least one selected from L-threonine and L-isoleucine.
